# EUROPEAN PATENT SPECIFICATION

(11) **EP 1 870 071 B1**
(45) Date of publication and mention of the grant of the patent: **26.12.2012**
(21) Application number: 06023926.6
(22) Date of filing: 17.11.2006
(51) Int. Cl.: A61J 7/00

(54) **Apparatus for detecting information of auxiliary tray and method thereof**
Detektionsgerät für einen zusätzlichen Schieber und Detektionmethode
Appareil de détection des informations contenues dans un tiroir auxiliaire et méthode de détection

(30) Priority: 21.06.2006 KR 20060055993; 20.09.2006 KR 20060091298
(43) Date of publication of application: 26.12.2007
(73) Proprietor: JVM Co., Ltd., Daegu 704-170 (KR)
(72) Inventor: Kim, Jun-ho, Dalseo-gu Daegu, 704-730 (KR)
(74) Representative: Grünecker, Kinkeldey, Stockmair & Schwanhäusser

(56) References cited:
- US-A1- 2003 057 231
- US-A1- 2005 125 097
- US-A1- 2006 058 917
- US-B1- 6 181 979
- US-B1- 6 799 685

## Description

### BACKGROUND OF THE INVENTION

### Field of the Invention

The present invention relates to detection of an auxiliary tray of an automatic medicine packaging machine, and more particularly, to an apparatus for detecting information of an auxiliary tray employed in a special tablet system as an apparatus used when manufacturing a half of a tablet or a special medicine that is not contained in a cassette (a medicine case) and a method thereof.

In more detail, the present invention relates to an auxiliary tray information detecting apparatus according to claim 1 automatically detecting information of an auxiliary tray, using a radio frequency identification (RFID) tag (a sticker type) attached to the auxiliary tray, without contact or using an electronic shelf label (ESL) system.

### Description of the Related Art

Humans are afraid of a great deal of diseases for throughout their life, and actually, frequently suffer from various diseases. Thus, efforts for treating various diseases continue and a variety of medicines for curing the diseases have appeared.

According to a kind of disease, some diseases can be cured with a single medicine. However, since human body is generally attacked with diseases for complex reasons and complex symptoms appear, a prescription is made by properly mixing several medicines according to the symptoms for the effective treatment of the diseases. These medicines are manufactured and supplied in commercial quantities in the form of a tablet or powder medicine for the convenience of prescription and use, and where necessary medicines are packaged by a dose according to the prescription of a medical doctor or a pharmacist and supplied to patients.

According a conventional packaging method of medicines, a sheet is folded to be overlapped and welded along the longitudinal direction by a regular pitch to continuously form pockets, necessary medicines are inserted into the respective pockets and openings of the pockets are sealed to package the medicines in the form of packs.

However, since the conventional medicine packaging method is almost always carried out manually by a pharmacist, a great deal of man power and time is required to compound the prescription. In order to solve this problem, an automatic packaging machine has been developed.

Generally, in the automatic medicine packaging machine, medicine is accommodated in cassettes according to a kind of the medicine, and tablets prescribed according to patient's diseases are automatically discharged to package every dose. In the automatic medicine packaging machine, plural cassettes in which a variety of tablets are accommodated are arranged in a plurality of frames, the cassettes are arranged in the frames so as to respectively discharge the prescribed tablets as required per dose down through respective chutes formed in the frames, the tablets discharged from the frames are collected in a hopper, and the tablets collected in the hopper are discharged down from the hopper to be sealed and packaged with packaging paper by a packaging device.

The conventional automatic medicine packaging machine includes a special tablet system (STS) used to compound a half of a tablet or special medicines that are not accommodated in the cassettes (medicine cases).

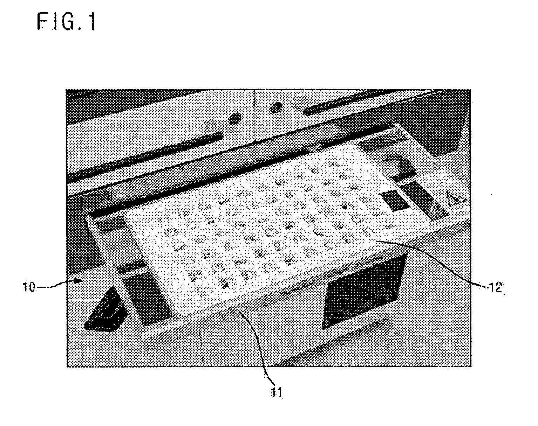

FIG. 1 is a schematic view illustrating the STS 10 employed in the conventional automatic medicine packaging machine.

The STS 10 includes a slide member 11 automatically opened according to manipulation of a button and manually closed, and a tray 12 placed on the slide member 11 to feed halves of the tablets and the special medicines that are not accommodated in the cassettes.

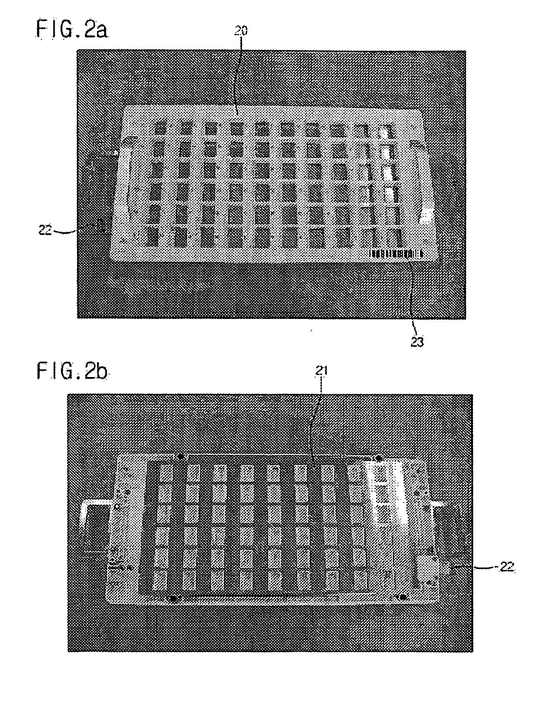

In order to arrange the medicines into the tray 12, an auxiliary tray 20, as shown in FIGS. 2a and 2b, is utilized to help the pharmacist to prepare the medicines to be compounded.

FIG. 2a illustrates the front side of the auxiliary tray 20 and FIG. 2b illustrates the rear side of the auxiliary tray 20.

The auxiliary tray 20 includes a sliding plate 21 and a lever 22 to slide the sliding plate 21. Moreover, the auxiliary tray 20 includes a barcode sticker 23 attached thereto to indicate information about the medicines accommodated in the auxiliary tray 20.

The pharmacist prepares the medicines to be compounded using the auxiliary tray 20 and carries the auxiliary tray 20 by hand, then pulls the lever 22 when the tray 12 of the STS 10 is aligned with the auxiliary tray 20 so that the medicines accommodated in the auxiliary tray 20 are placed at respective corresponding positions of the tray 12.

After that, when the pharmacist presses a rearward moving button of the STS, the slide member 11 automatically enters the STS. Hereinafter, the medicines are automatically fed by a controller of the STS and automatically packaged by following processes.

Meanwhile, the conventional auxiliary tray 20 has no function of storing information about the patient and the medicines, but provides the information of the medicines prepared in the corresponding auxiliary tray 20 on a barcode sticker that is attached to the front side of the auxiliary tray 20 and in which the simple information of the medicines is recorded. When detecting the information of the medicines prepared in the auxiliary tray 20, a user (the pharmacist) manually scans the barcode sticker 23 attached to the front side of the auxiliary tray 20 with a barcode reader to read the information recorded in the barcode sticker 23 so that the information of the medicines can be detected.

Thus, in this case, since the barcode stickers must be manufactured according to information of respective medicines, it is inconvenient to attach the manufactured barcodes to the corresponding auxiliary trays.

Moreover, since the barcode sticker is manufactured to be disposable, when the auxiliary tray is to be used for other medicines, it is inconvenient to detach the previously attached barcode from the auxiliary tray and to attach a newly manufactured barcode to the auxiliary tray.

In addition, when the user reads the information of the medicines prepared in the auxiliary tray, the user feels some inconvenience to manually scan the information with the barcode reader.

Particularly, in the case of detecting the information of the medicines with the barcode reader, the user must carefully pay attention to confirm the information of the medicines

### SUMMARY OF THE INVENTION

Therefore, the present invention has been made in view of the above and/or other problems occurring when using the auxiliary tray employed in the conventional special tablet system, and it is an aspect of the present invention to provide an auxiliary tray information detecting apparatus in which a radio frequency identification (RFID) tag (a sticker type) is attached to the auxiliary tray that is employed in the special tablet system, there is an apparatus used to compound the prescription with halves of tablets and/or special medicines not contained in a cassette (or a medicine case) and information about the auxiliary tray is automatically detected without contact with the RFID tag, and a method thereof.

It is another aspect of the present invention to provide an auxiliary information detecting apparatus in which a radio frequency identification (RFID) is attached to the auxiliary tray for detecting information of the auxiliary tray without contact with the auxiliary tray and information about a medicine and a patient is enabled to be stored and modified so that inconvenience from use of a conventional disposable barcode type sticker can be solved, and a method thereof.

It is another aspect of the present invention to provide an auxiliary tray information detecting apparatus in which a great deal of information can be stored by employing a radio frequency identification (RFID) tag to the auxiliary tray for detecting the information without contact with the RFID tag, and a method thereof.

It is yet another aspect of the present invention to provide an auxiliary tray information detecting apparatus in which an indicating device such as a price card, a liquid crystal display, a flexible numeric display (NFD), a dot matrix display, or the like is attached to the auxiliary tray and information about a patient and a medicine that is contained in the auxiliary tray is displayed in real time using an electronic shelf label (ESL) system (an indicating unit manager) so that a user (a prescription manager) can easily confirm the information about the auxiliary tray.

In accordance with the present invention, the above and other objects can be accomplished by the provision of an apparatus for detecting information of an auxiliary tray to supply medicines to a tray that is installed on a slide member provided in a special tablet system used when compounding a half of a tablet or a medicine not contained in a cassette, the apparatus including: a radio frequency identification tag, installed on a predetermined position of the auxiliary tray, in which medicine information and patient information are recorded; a radio frequency identification information reader to read the information recorded in the radio frequency identification tag without contact; a commercial terminal to compare the medicine information and the patient information read by the radio frequency identification information reader with prescription information inputted from the exterior, to determine the auxiliary tray as a predetermined auxiliary tray when the medicine information and the patient information is identical to the prescription information, and to generate an alarm for preventing a prescription error when the medicine information and the patient information is different from the prescription information; and an alarm device connected to the commercial terminal to generate the alarm for preventing the prescription error.

The radio frequency identification information reader is installed on a predetermined position of the slide member, and includes a radio frequency identification reader to read data stored in the radio frequency identification tag without contact, a central processing unit to control a transmission of the data read by the radio frequency identification reader, and a transceiver to transmit radio frequency identification reading information outputted from the central processing unit to an external device using a predetermined communication protocol.

The commercial terminal includes: a transceiver to receive auxiliary tray reading information transmitted from the radio frequency identification information reader; a controller to compare the auxiliary tray reading information transmitted through the transceiver with the previously stored prescription information and to generate the alarm for preventing the prescription error when the auxiliary tray reading information is different from the previously stored prescription information; a display to display the prescription information and the auxiliary tray reading information on a screen; a memory in which the prescription information and a program for comparing information and generating the alarm are stored; and an alarming unit to generate an alarming signal corresponding to a controlling signal when the controlling signal is outputted from the controller and to transmit the alarming signal to the alarm device.

The controller includes: an interface to interface between the transceiver, the display, the alarming unit, the memory, and external device for data interface; an information comparator to compare the prescription information with the auxiliary tray reading information; an alarm generator to generate an alarm control signal when the prescription information is different from the auxiliary tray reading information as a result of the comparison by the information comparator; and a display controller to control the displaying of the prescription information and the auxiliary tray reading information.

Here, the alarm device is installed at a predetermined position of the slide member, includes a light emitting diode to emit light according to an inputted alarm signal so as to generate a visual alarm warning of the prescription error, or a device to provide an alarm warning of the prescription error by generating an alarming sound according to an inputted audible alarm signal.

Moreover, a variety of alarming information can be displayed by a display of a commercial terminal.

In accordance with another aspect of the present invention, there is provided an apparatus for detecting information of an auxiliary tray to supply medicines to a tray that is installed on a slide member provided in a special tablet system used when compounding a half of a tablet or a medicine not contained in a cassette, the apparatus including: an indicating unit installed at a predetermined position of the auxiliary tray to visually indicate medicine information and patient information; and an indicating unit managing unit connected to the indicating unit through a POS system to transmit the medicine information of the medicines accommodated in the auxiliary tray and the patient information to the indicating unit.

The indicating unit includes: a radio communication unit to receive indicating information such as the medicine information and the patient information that are transmitted from the indicating unit managing unit through radio communication and to transmit confirming information to the indicating unit managing unit as needed; a signal processor to remove noise and extract a pure data signal from a signal received by the radio communication unit and to convert the extracted pure data signal into a digital signal; a controller to perform authorization/analysis of the signal processed by the signal processor and to control now-indicated information to be modified when the processed signal is determined to be a signal of the controller as a result of the authorization/analysis; a memory connected to the controller to store a program for the authorization/analysis of information and the indicated information; an information modifier connected to the controller to modify the indicated information; and an indicator connected to the information modifier to indicate the auxiliary tray information according to an output from the information modifier.

The indicating unit includes one of a price card in which information to be displayed can be modified, a liquid crystal display, a flexible numeric display, and a dot matrix display.

The indicating unit managing unit includes: an input unit to input information to be recorded and modified information of the information to be recorded to the indicating unit attached to the auxiliary tray; a communication unit to receive recording information and the modified information of the respective indicating units in real time through the communication with the POS system; a central processing unit connected to the input unit and the communication unit to transmit the indicating information to the respective indicating units and to control the transmission of the information for the modification of the information to be indicated; a display connected to the central processing unit to display the information to be transmitted to a specific indicating unit; and a radio communication unit connected to the central processing unit to transmit the indicating information to the indicating unit via radio communication.

In accordance with another aspect of the present invention, there is provided a method of detecting information of an auxiliary tray including: performing an initialization of an apparatus when electric power is supplied and checking whether prescription information is received; storing the received prescription information in the memory when the prescription information is received as a result of the checking; determining whether auxiliary tray reading information is received after storing the prescription information; comparing the received auxiliary tray reading information with the stored prescription information when the auxiliary tray reading information is received; and displaying a status when the auxiliary tray reading information is identical to the prescription information, and a status when the auxiliary tray reading information is different from the prescription information so as to generate an alarm for preventing a prescription error.

### BRIEF DESCRIPTION OF THE DRAWINGS

These and/or other objects and advantages of the present invention will become apparent and more readily appreciated from the following description of the embodiments, taken in conjunction with the accompanying drawings, in which:
Fig. 1 is a schematic view illustrating a special tablet system employed in a conventional automatic medicine packaging machine;
FIGS. 2a and 2b are perspective views illustrating an auxiliary tray employed in the special tablet system in FIG. 1, in which FIG. 2a is a front perspective view of the auxiliary tray and FIG. 2b is a rear perspective view thereof;
FIG. 3 is a block diagram illustrating an apparatus for detecting information of an auxiliary tray according to a first embodiment of the present invention;
FIG. 4 is a flowchart illustrating a method of detecting information of an auxiliary tray according to an embodiment of the present invention;
FIG. 5 is a perspective view illustrating the auxiliary tray having an indicating unit for detecting the information of the auxiliary tray in the present invention;
FIG. 6 is a block diagram illustrating an apparatus for detecting information of an auxiliary tray according to a second embodiment of the present invention; and
FIG. 7 is a block diagram illustrating an auxiliary tray information indicating device of the apparatus for detecting information in FIG. 6.

### DESCRIPTION OF THE PREFERRED EMBODIMENTS

Hereinafter, an apparatus for detecting information of an auxiliary tray and a method thereof according to embodiments of the present invention will be described in detail with reference to the accompanying drawings. In the following description of the present invention, if the detailed description of the already known structure and operation may confuse the subject matter of the present invention, the detailed description thereof will be omitted.

### Embodiment 1

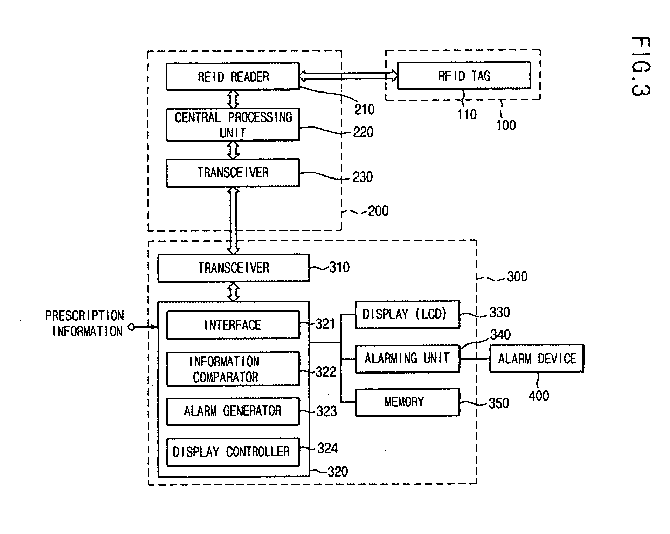

FIG. 3 is a block diagram illustrating an apparatus for detecting information of an auxiliary tray according to a first embodiment of the present invention.

Here, a reference numeral 100 is assigned to an auxiliary tray to which a radio frequency identification (RFID) tag 110 in which information of medicines and information of a patient are recorded is attached, and the information of the medicines and the patient recorded in the RFID tag 110 can be modified.

A reference numeral 200 is assigned to a RFID information reader to read the information recorded in the RFID tag 110 attached to the auxiliary tray 100 without contact with the RFID tag 110, and preferably the RFID information reader 200 includes an RFID reader 210 to read data stored in the RFID tag 110 without contact with the RFID tag 110, a central processing unit (CPU) 220 to control the transmission of the information (data) read from the RFID reader 210, and a transceiver 230 to transmit the RFID reading information outputted from the CPU 220 to an external device using a predetermined protocol. Here, the RFID information reader 200 is installed at a predetermined position of the slide member (indicated by 11 in FIG. 1).

A reference numeral 300 is assigned to a commercial terminal (a personal computer, PC) to compare the information of the medicines and the patient read by the RFID information reader 200 with information of the prescription inputted from the exterior, to determine a tray to which the RFID tag is attached as a predetermined auxiliary tray 100 when the information of the medicines and the patient is identical to the information of the prescription, and to generate an alarm for preventing a prescription error when the information of the medicines and the patient is different from the information of the prescription. The commercial terminal 300 preferably includes a transceiver 310 to receive the reading information (data) of the auxiliary tray transmitted from the RFID information reader 200, a controlling unit 320 to compare the reading information of the auxiliary tray 100 transmitted from the transceiver 310 with the previously stored prescription information and to generate the alarm when the reading information of the auxiliary tray 100 is different from the prescription information, a display (LCD) 330 to display the prescription information and the reading information of the auxiliary tray 100 on a screen, a memory 350 in which the prescription information is stored and a program to carry out the comparison of the information and to generate the alarm is stored, and an alarming unit 340.

Moreover, the controlling unit 320 preferably includes an interface 321 to interface the transceiver 310, the display 330, the alarming unit 340, the memory 350, and an external device for the data interface, an information comparator 322 to compare the prescription information with the reading information of the auxiliary tray 100, an alarm generator 323 to generate an alarm control signal when the prescription information is different from the reading information of the auxiliary tray 100 as a result of the comparison by the information comparator 322, and a display controller 324 to control the displaying of the prescription information and the reading information of the auxiliary tray 100.

A reference numeral 400 is assigned to an alarm device connected to the commercial terminal 300 to generate an alarm for preventing the prescription error, and preferably connected to a predetermined position of the slide member 11. In addition, the alarm device 400 includes a light emitting diode (LED) to emit light according to the alarm signal outputted from the alarming unit 340 to generate a visual alarm warning of the prescription error, or a device to generate an alarming sound according to the alarm signal outputted from the alarming unit 340 to generate an audio alarm warning of the prescription error.

The apparatus for detecting information of an auxiliary tray, constructed as described above according to the present invention, when the user (the pharmacist) prepares medicines to be provided to a specific patient in the auxiliary tray 100 and the preparation is completed, records data such as information about the medicines prepared in the auxiliary tray 100 and a patient to be provided with the medicines in the RFID tag 110 with a separate RFID reader (not shown). Here, the information of the patient and the information of the medicines, which are stored in the RFID tag 110, can be modified as needed. The RFID tag 110 may be attached to any position of the auxiliary tray 100, and more preferably to a place with which the user's body does not interfere when the user carries the auxiliary tray 100 by grasping grips of the auxiliary tray 100 and the auxiliary tray 100 is positioned on a tray 12 of a special tablet system (STS) 10. Without a need of attaching a single RFID tag to every auxiliary tray and replacing the attached RFID tag when the information of the patent and the information of the medicines are changed later, the information of the patient and the information of the medicines are modified by the RFID reader so that it is very convenient to use in comparison to the conventional barcode sticker. Moreover, when the RFID tag is used, the inconvenience of the conventional barcode sticker, that is, of replacing the barcode sticker whenever the information of the medicines and the information of the patient are changed, can be solved.

Next, when the user manually carries the auxiliary tray 100 to which the RFID in which the information of the patient and the information of the medicines are recorded is attached and the auxiliary tray 100 is positioned at a position near a predetermined position of the tray 12 positioned on the slide member 11 in the STS 10 as shown in FIG. 1, the RFID reader 210 of the RFID information reader 200 installed at a predetermined position of the slide member 11 reads the information of the patient and the information of the medicines recorded in the RFID tag 110 without contact with the RFID tag 110. The read information of the auxiliary tray 100 is transmitted to the commercial terminal 300 through the transceiver 230 by the CPU 220.

The controlling unit 320 receives the prescription information from the exterior through the interface 321 and stores the received prescription information to the memory 350. After storing the prescription information to the memory 350, when the information (information about the patient and the medicines) of the auxiliary tray 100 is received from the RFID information reader 200 through the transceiver 310, the display controller 324 displays the information on the display 330, and the information comparator 322 compares the prescription information stored in the memory 350 with the information of the auxiliary tray 100. As a result of the comparison, when the information is identical to each other, this status is displayed on the display 330 and following operations are carried out. On the other hand, when the stored prescription information is different from the received information of the auxiliary tray 100, the alarm generator 323 provides the alarm control signal to generate an alarm warning of the prescription error to the alarming unit 340 for the prevention of the prescription error, and the display controller 324 displays the status of disagreement of the information on the display 330. Here, the interface 321, the information comparator 322, the alarm generator 323, and the display controller 324 of the controlling unit 320 are not depicted in the drawings, but substantially are implemented to carry out an internal communication. Next, when the alarm control signal is inputted, the alarming unit 340 provides the alarm signal to the alarm device 400 so as to generate an alarm warning of the prescription error.

Then, the alarm device 400 transmits a visual alarm or an audio alarm so that the user (the pharmacist) using the auxiliary tray 100 can perceive that the auxiliary tray 100 is erroneously placed on the tray 12 at the present time. Here, the alarm device 400 may be positioned at any position of the STS 10, and may be preferably implemented by an LED for visually alarming or an alarm device such as a buzzer, etc. for audibly alarming. Meanwhile, since the status when the alarm device 400 is operated is a case when an auxiliary tray 100 in which medicines for another patient different from the prescription information are prepared is placed on the STS 10, this status is immediately alarmed to the user so that the user can easily perceive this status. The conventional technique is inconvenient because the user must manually confirm the information of the barcode sticker attached to the auxiliary tray with the barcode reader to detect the information of the auxiliary tray and as such the precision is deteriorated. On the other hand, since the apparatus for detecting information of an auxiliary tray automatically detects the information of the auxiliary tray without contact, the prescription error can be prevented and the information of the auxiliary tray can be effectively detected.

### Embodiment 2

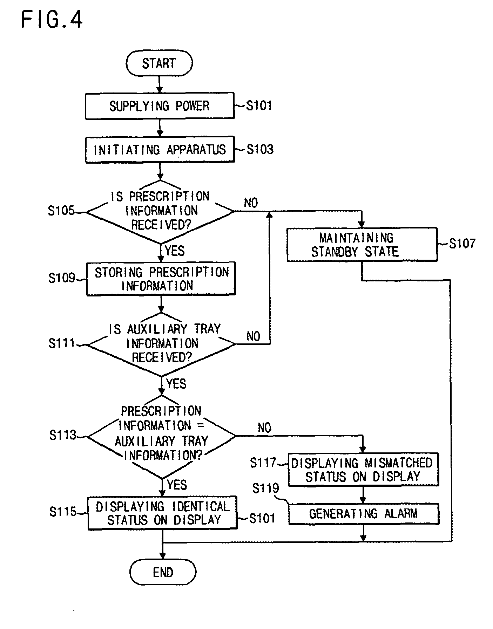

FIG. 4 is a flowchart illustrating a "method of detecting information of an auxiliary tray" according to an embodiment of the present invention.

As shown in the drawing, the method includes performing an initialization of the auxiliary tray information detecting apparatus and checking whether the prescription information is received (S101 to S105), maintaining a standby status when the prescription information is not received as a result of the checking (S107), storing the received prescription information in the memory when the prescription information is received as a result of the checking (S109), determining whether the reading information of the auxiliary tray is received after storing the prescription information (S111), comparing the received reading information of the auxiliary tray with the stored prescription information when the reading information of the auxiliary tray is received (S113), and displaying a status when the reading information of the auxiliary tray is identical to the prescription information, displaying a status when the reading information of the auxiliary tray is not identical to the prescription information, and generating an alarm for preventing the prescription error (S115 to S119).

The method of detecting the information of the auxiliary tray according to the present invention, constructed as described above, firstly carries out overall operation of the apparatus in the process S103 when a driving power is supplied to the apparatus in the process S101, and checks whether the prescription information is received or not in the process S105.

As a result of the checking, when the prescription information is not received, the standby status is maintained (S107), and on the other hand, when the prescription information is received, the received prescription information is stored in the memory 350 in the process S109.

Next, in the process S111, whether the information of the auxiliary tray is received or not is checked. If the information of the auxiliary tray is not received, the present status is maintained, and the process S113 is carried out when the information of the auxiliary tray is received and the prescription information previously stored in the memory is compared with the information of the auxiliary tray.

As a result of the comparison, when the prescription information is identical to the information of the auxiliary tray, the status is displayed on the display in the process S115 and the following operation is carried out. On the other hand, when the prescription information is different from the information of the auxiliary tray, the status when the prescription information is different from the information of the auxiliary tray is displayed on the display in the process S117, and an alarm is generated visually or audibly in the process S119 such that the user (the pharmacist) can easily perceive the status. By doing so, the prescription error that may occur can be prevented.

### Embodiment 3

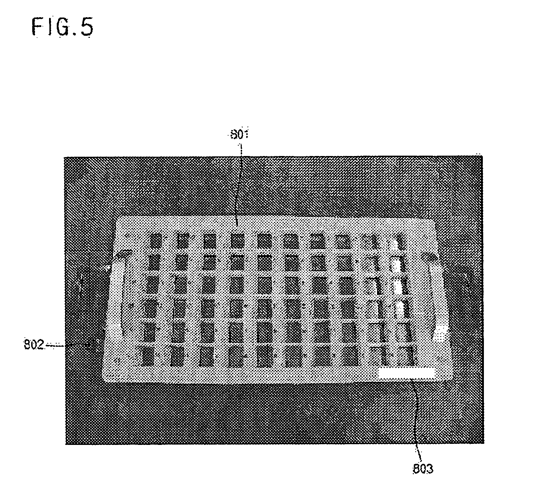

FIG. 5 is a perspective view illustrating the front side of an auxiliary tray employing an indicating unit according to the present invention. The auxiliary tray 801 includes a slide plate and a lever 802 to move the slide plate. An indicating unit 803 to indicate the information of medicines accommodated in the auxiliary tray 801 and the patient information is attached to a side of the auxiliary tray 801.

When the medicines to be compounded are prepared in the auxiliary tray 801 and the information is transmitted to the indicating unit 803 through an indicating unit managing unit 500, the indicating unit 803 indicates the information of the medicines and the patient information so that a pharmacist or other people can easily perceive the information of the medicines prepared in the auxiliary tray 801 and whom the prescribed medicine has been compounded for.

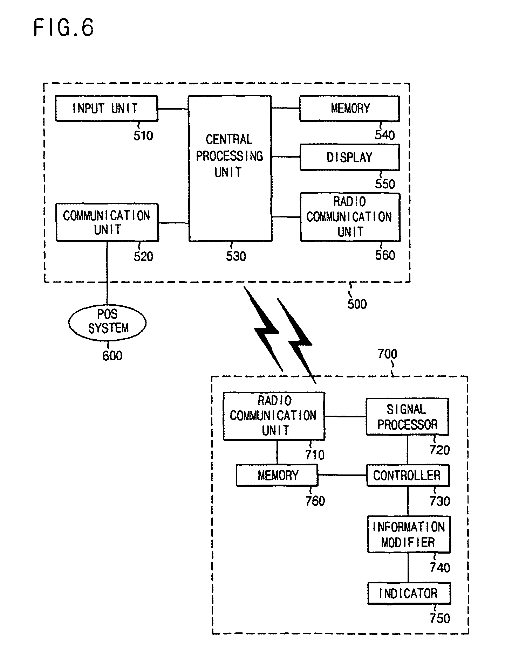

FIG. 6 is a block diagram illustrating an "apparatus for detecting information of an auxiliary tray" according to a second embodiment of the present invention. The apparatus includes an indicating unit 700, installed in an auxiliary tray 801, in which information of medicines and patient information are recorded, and an indicating unit managing unit 500 connected to the indicating unit 700 through a POS system 600 and transmitting the information of the medicines accommodated in the auxiliary tray 801 and the patient information to the indicating unit 700.

Here, the indicating unit 700 includes a radio communication unit 710 to receive the indicating information such as the medicine information and the patient information that are transmitted from the indicating unit managing unit 500 and to transmit confirming information to the indicating unit managing unit 500 as needed, and a signal processor 720 connected to the radio communicating unit 710 to remove noise from the received signal and to extract a pure data signal from the received signal and convert the extracted pure data signal into a digital signal. The indicating unit 700 further includes a controller 730 to perform authorization/analysis of the signal processed by the signal processor 720 and to control now-indicated information to be modified when the processed signal is determined to be a signal of the controller 730 as a result of the authorization/analysis, a memory 760 connected to the controller 730 to store a program for the authorization/analysis of the information and the indicated information, an information modifier 740 connected to the controller 730 to modify the indicated information, and an indicator 750 connected to the information modifier 740 to indicate the auxiliary tray information (the medicine information and the patient information).

The indicating unit managing unit 500 is one of electronic shelf label (ESL) systems, and includes an input unit 510 to input information to be recorded and modified information of the information to be recorded to the indicating units 700 and 803 attached to the auxiliary tray 801, a communication unit 520 to receive the recording information and the modified information of the respective indicating units in real time through the communication with the POS system 600, a central processing unit 530 connected to the input unit 510 and the communication unit 520 to transmit the indicating information and to control the transmission of the information for the modification of the information to be indicated, a display 550 connected to the central processing unit 530 to display the information to be transmitted to a specific indicating unit, a radio communication unit 560 connected to the central processing unit 530 to transmit the indicating information to the indicating unit 700 via a radio communication, and a memory 540 connected to the central processing unit 530 to store a controlling program and generated data.

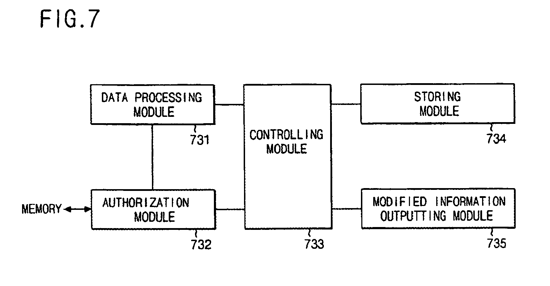

The "apparatus for detecting information of an auxiliary tray", constructed as described above, according to the second embodiment of the present invention will be described with reference to FIGS. 5 to 7 as follows.

Firstly, as shown in FIG. 5, when the indicating unit 803 to which the patient information and the medicine information are recorded is placed at a predetermined position of the auxiliary tray 801, the pharmacist or other people can easily perceive which medicines are prepared in the auxiliary tray 801 and for whom of the patients they are prepared by seeing only the information in the indicating unit 803.

Here, the information indicated by the indicating unit 803 that is installed to the auxiliary tray 801 can be modified in real time.

In more detail, when a specific auxiliary tray is selected and the information to be indicated by the indicating unit 700 attached to the auxiliary tray or modified information is inputted by the input unit 510, the central processing unit 530 of the indicating unit managing unit 500 displays the selected auxiliary tray and the auxiliary tray information on the display 550 such that a person to modify the information can easily check whether the modified information is correct or not.

If correct, the indicating information is transmitted by the radio communication unit 560 to the indicating unit 700. On the other hand, in the indicating unit 700, the radio communication unit 710 receives the indicating information, the signal processor 720 processes the received signal through a signal processing such as the removal of noise, the data extraction, and the conversion from an analog signal into a digital signal and supplies the processed signal to the controller 730.

In the controller 730, a data processing module 731 separates an identification number from data by processing the received data and an authorization module 732 compares the separated identification number with an identification number stored in the memory 760 to perform the authorization.

If the data is a controlling module's 733 own data, the controlling module 733 stores the received data in the memory 760 through a storing module 734 and outputs information (the medicine information and the patient information) to be modified through a modified information outputting module 735.

The information modifier 740 converts the modified information outputted from the modified information outputting module 735 into displaying data and provides the converted displaying data to the indicator 750 so that the indicator 750 replaces the currently displayed information with the modified information to be displayed.

Here, the indicating unit 700 visually displays the medicine information and the patient information and is one of a price card in which information to be displayed can be modified, an LCD, a flexible numeric display (FND), and a dot matrix display.

As described above, although the third embodiment of the present invention has been described by only a case that a single indicating unit managing unit is connected to a single indicating unit, substantially the present invention has a type in which a single indicating unit managing unit is connected to plural indicating units. Thus, since the plural indicating units almost always display information different from each other, the auxiliary trays are distinguished using the identification numbers. Since the distinguishment of the auxiliary trays using the identification numbers is already known to those skilled in the art as a conventional technique, its detailed description will be omitted.

According to the present invention, the RFID tag (a sticker type) is attached to the auxiliary tray and the RFID information reader is installed to the slide member of the STS so that the information of the auxiliary tray can be automatically detected without contact.

Moreover, the RFID tag for the contactless detection is employed to the auxiliary tray so that the inconvenience occurring when the auxiliary tray information is manually detected as the conventional art can be solved.

In addition, the detection of the auxiliary tray information is not carried out manually but by data comparison without contact, so that the precision of the information detection can be improved and the prescription error caused by erroneous detection of the auxiliary tray can be prevented.

Moreover, the electronic shelf label (ESL) system is employed so that the information of the auxiliary tray can be easily detected.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A special tablet system comprising:
a slide member;
a tray installed on the slide member;
an auxiliary tray for supplying medicines to the tray that is installed on the slide member; and
an apparatus configured to detect information of the auxiliary tray, the apparatus comprising:
a radio frequency identification tag, installed on a predetermined position of the auxiliary tray, in which medicine information and patient information are recorded;
a radio frequency identification information reader configured to read the information recorded in the radio frequency identification tag without contact; a commercial terminal configured to compare the medicine information and the patient information read by the radio frequency identification information reader with prescription information inputted from the exterior, the commercial terminal further configured to determine the auxiliary tray as a predetermined auxiliary tray when the medicine information and the patient information is identical to the prescription information, and configured to generate an alarm for preventing a prescription error when the medicine information and the patient information is different from the prescription information; and
an alarm device connected to the commercial terminal configured to generate the alarm for preventing the prescription error.

2. The apparatus according to claim 1, wherein the radio frequency identification tag enables the modification of the medicine information and the patient information recorded thereto.

3. The apparatus according to claim 1, wherein the radio frequency identification information reader comprises:
a radio frequency identification reader to read data stored in the radio frequency identification tag without contact;
a central processing unit to control a transmission of the data read by the radio frequency identification reader; and
a transceiver to transmit radio frequency identification reading information outputted from the central processing unit to an external device using a predetermined communication protocol.

4. The apparatus according to claims 1 or 3, wherein the radio frequency identification information reader is installed on a predetermined position of the slide member.

5. The apparatus according to claim 1, wherein the commercial terminal comprises:
a transceiver to receive auxiliary tray reading information transmitted from the radio frequency identification information reader;
a controller to compare the auxiliary tray reading information transmitted through the transceiver with the previously stored prescription information and to generate the alarm for preventing the prescription error when the auxiliary tray reading information is different from the previously stored prescription information;
a display to display the prescription information and the auxiliary tray reading information on a screen;
a memory in which the prescription information and a program for comparing information and generating the alarm are stored; and
an alarming unit to generate an alarming signal corresponding to a controlling signal when the controlling signal is outputted from the controller and to transmit the alarming signal to the alarm device.

6. The apparatus according to claim 5, wherein the controller comprises:
an interface to interface between the transceiver, the display, the alarming unit, the memory, and external device for data interface;
an information comparator to compare the prescription information with the auxiliary tray reading information;
an alarm generator to generate an alarm control signal when the prescription information is different from the auxiliary tray reading information as a result of the comparison by the information comparator; and
a display controller to control the displaying of the prescription information and the auxiliary tray reading information.

7. The apparatus according to claim 6, wherein the alarm device is installed at a predetermined position of the slide member.

8. The apparatus according to claims 1 or 7, wherein the alarm device comprises a light emitting diode to emit light according to an inputted alarm signal so as to generate a visual alarm warning of the prescription error.

9. The apparatus according to claims 1 or 7, wherein the alarm device comprises a device to provide an alarm warning of the prescription error by generating an alarming sound according to an inputted audible alarm signal.

10. A method of detecting information of an auxiliary tray to supply medicines to a tray that is installed on a slide member provided in a special tablet system used when compounding a half of a tablet or a medicine not contained in a cassette, the method comprising:
performing an initialization of an apparatus when electric power is supplied and checking whether prescription information is received;
storing the received prescription information in the memory when the prescription information is received as a result of the checking;
determining whether auxiliary tray reading information is received after storing the prescription information;
comparing the received auxiliary tray reading information with the stored prescription information when the auxiliary tray reading information is received; and
displaying a status when the auxiliary tray reading information is identical to the prescription information, and a status when the auxiliary tray reading information is different from the prescription information so as to generate an alarm for preventing a prescription error.

11. An apparatus detecting information of an auxiliary tray to supply medicines to a tray that is installed on a slide member provided in a special tablet system used when compounding a half of a tablet or a medicine not contained in a cassette, the apparatus comprising:
an indicating unit installed at a predetermined position of the auxiliary tray to visually indicate medicine information and patient information; and
an indicating unit managing unit connected to the indicating unit through a POS system to transmit the medicine information of the medicines accommodated in the auxiliary tray and the patient information to the indicating unit.

12. The apparatus according to claim 11, wherein the indicating unit comprises:
a radio communication unit to receive indicating information such as the medicine information and the patient information that are transmitted from the indicating unit managing unit through radio communication and to transmit confirming information to the indicating unit managing unit as needed;
a signal processor to remove noise and extract a pure data signal from a signal received by the radio communication unit and to convert the extracted pure data signal into a digital signal;
a controller to perform authorization/analysis of the signal processed by the signal processor and to control now-indicated information to be modified when the processed signal is determined to be a signal of the controller as a result of the authorization/analysis;
a memory connected to the controller to store a program for the authorization/analysis of information and the indicated information;
an information modifier connected to the controller to modify the indicated information; and
an indicator connected to the information modifier to indicate the auxiliary tray information according to an output from the information modifier.

13. The apparatus according to claims 11 or 12, wherein the indicating unit comprises one of a price card in which information to be displayed can be modified, a liquid crystal display, a flexible numeric display, and a dot matrix display.

14. The apparatus according to claim 12, wherein the controller comprises:
a data processing module to separate an identification number from data by processing the received data;
an authorization module compares the separated identification number with an identification number stored in the memory to perform the authorization;
a controlling module to control storing of the received data through a storing module and to output information to be modified when the data is determined to be data of the controlling module as a result of the authorization; and
a modified information outputting module to output the medicine information and the patient information to be modified in response to the control of the controlling module.

15. The apparatus according to claim 11, wherein the indicating unit managing unit comprises:
an input unit to input information to be recorded and modified information of the information to be recorded to the indicating unit attached to the auxiliary tray;
a communication unit to receive recording information and the modified information of the respective indicating units in real time through the communication with the POS system;
a central processing unit connected to the input unit and the communication unit to transmit the indicating information to the respective indicating units and to control the transmission of the information for the modification of the information to be indicated;
a display connected to the central processing unit to display the information to be transmitted to a specific indicating unit; and
a radio communication unit connected to the central processing unit to transmit the indicating information to the indicating unit via radio communication.

## Patentansprüche

1. Sondertabletten-System, das umfasst:
ein Schubladenelement;
eine Kassette, die an dem Schubladenelement installiert ist;
eine Hilfskassette zum Zuführen von Medikamenten zu der Kassette, die an dem Schubladenelement installiert ist; und
eine Vorrichtung, die so eingerichtet ist, dass sie Informationen der Hilfskassette erfasst,
wobei die Vorrichtung umfasst:
ein RFID-Etikett (radio frequency identification tag), das an einer vorgegebenen Position der Hilfskassette installiert ist und auf dem Medikamenten-Informationen und Patienten-Informationen aufgezeichnet sind;
eine Einrichtung zum Lesen von RFI-Informationen, die so eingerichtet ist, dass sie die auf dem RFID-Etikett aufgezeichneten Informationen kontaktlos liest;
ein Verkaufs-Terminal, das so eingerichtet ist, dass es die durch die Einrichtung zum Lesen von RFID-Informationen gelesenen Medikamenten-Informationen und Patienten-Informationen mit Verschreibungs-Informationen vergleicht, die von außen eingegeben werden, wobei das Verkaufs-Terminal des Weiteren so eingerichtet ist, dass es die Hilfskassette als eine vorgegebene Hilfskassette bestimmt, wenn die Medikamenten-Informationen und die Patienten-Informationen identisch mit den Verschreibungs-Informationen sind, und so eingerichtet ist, dass es eine Warnung erzeugt, um einen Verschreibungs-Fehler zu verhindern, wenn sich die Medikamenten-Informationen und die Patienten-Informationen von den Verschreibungs-Informationen unterscheiden; und
eine Warneinrichtung mit dem Verkaufs-Terminal verbunden ist und so eingerichtet ist, dass sie die Warnung zum Verhindern des Verschreibungs-Fehlers erzeugt.

2. Vorrichtung nach Anspruch 1, wobei das RFID-Etikett die Modifizierung der darauf aufgezeichneten Medikamenten-Informationen und Patienten-Informationen ermöglicht.

3. Vorrichtung nach Anspruch 1, wobei die Einrichtung zum Lesen von RFID-Informationen umfasst:
eine RFID-Leseeinrichtung, die auf dem RFID-Etikett gespeicherte Daten kontaktlos liest;
eine zentrale Verarbeitungseinheit, die eine Übertragung der durch die RFID-Leseeinrichtung gelesenen Daten steuert; und
einen Sendeempfänger, der von der zentralen Verarbeitungseinheit ausgegebene RFID-Lese-Informationen unter Verwendung eines vorgegebenen Kommunikationsprotokolls zu einer externen Einrichtung sendet.

4. Vorrichtung nach Anspruch 1 oder 3, wobei die Einrichtung zum Lesen von RFID-Informationen an einer vorgegebenen Position des Schubladenelementes installiert ist.

5. Vorrichtung nach Anspruch 1, wobei das Verkaufs-Terminal umfasst:
einen Sendeempfänger zum Empfangen von der Einrichtung zum Lesen von RFID-Informationen gesendeter Lese-Informationen der Hilfskassette;
eine Steuereinrichtung, die die über den Sendeempfänger gesendeten Lese-Informationen der Hilfskassette mit bereits gespeicherten Verschreibungs-Informationen vergleicht und die Warnung zum Verhindern des Verschreibungs-Fehlers erzeugt, wenn sich die Lese-Informationen der Hilfskassette von den bereits gespeicherten Verschreibungsinformationen unterscheiden;
eine optische Anzeigeeinrichtung zum optischen Anzeigen der Verschreibungs-Informationen und der Lese-Informationen der Hilfskassette auf einem Bildschirm;
einen Speicher, in dem die Verschreibungs-Informationen sowie ein Programm zum Vergleichen von Informationen und zum Erzeugen der Warnmeldung gespeichert sind; und
eine Warneinheit, die ein Warnsignal entsprechend einem Steuersignal erzeugt, wenn das Steuersignal von der Steuereinrichtung ausgegeben wird, und das Warnsignal zu der Warneinrichtung sendet.

6. Vorrichtung nach Anspruch 5, wobei die Steuereinrichtung umfasst:
eine Schnittstelle, die Verbindung zwischen dem Sendeempfänger, der optischen Anzeigeeinrichtung, der Warneinheit, dem Speicher und einer externen Einrichtung für Datenverbindung herstellt;
eine Informations-Vergleichseinrichtung zum Vergleichen der Verschreibungs-Informationen mit den Lese-Informationen der Hilfskassette;
eine Warnungs-Erzeugungseinrichtung, die ein Warn-Steuersignal erzeugt, wenn der Vergleich durch die Informations-Vergleichseinrichtung ergibt, dass sich die Verschreibungs-Informationen von den Lese-Informationen der Hilfskassette unterscheiden; und eine Steuereinrichtung der optischen Anzeige zum Steuern des optischen Anzeigens der Verschreibungs-Informationen und der Lese-Informationen der Hilfskassette.

7. Vorrichtung nach Anspruch 6, wobei die Warneinrichtung an einer vorgegebenen Position des Schubladenelementes installiert ist.

8. Vorrichtung nach Anspruch 1 oder 7, wobei die Warneinrichtung eine Leuchtdiode umfasst, die Licht entsprechend einem eingegebenen Warnsignal emittiert, um so eine optische Warnung zu erzeugen, die auf den Verschreibungsfehler hinweist.

9. Vorrichtung nach Anspruch 1 oder 7, wobei die Warneinrichtung eine Einrichtung umfasst, die eine Warnung bereitstellt, die auf den Verschreibungs-Fehler hinweist, indem sie einen Warnton entsprechend einem eingegebenen akustischen Warnsignal erzeugt.

10. Verfahren zum Erfassen von Informationen einer Hilfskassette zum Zuführen von Medikamenten zu einer Kassette, die an einem Schubladenelement installiert ist, das in einem Sondertabletten-System vorhanden ist, das eingesetzt wird, wenn eine Hälfte einer Tablette oder ein Medikament zusammengestellt wird, das nicht in einem Behälter enthalten ist, wobei das Verfahren umfasst:
Durchführen einer Initialisierung einer Vorrichtung, wenn Strom zugeführt wird, und Prüfen, ob Verschreibungs-Informationen empfangen werden;
Speichern der empfangenen Verschreibungs-Informationen in dem Speicher, wenn das Prüfen ergibt, dass Verschreibungs-Informationen empfangen werden;
Feststellen, ob Lese-Informationen der Hilfskassette empfangen werden, nachdem die Verschreibungs-Informationen gespeichert worden sind;
Vergleichen der empfangenen Lese-Informationen der Hilfskassette mit den gespeicherten Verschreibungs-Informationen, wenn die Lese-Informationen der Hilfskassette empfangen werden; und
optisches Anzeigen eines Status, wenn die Lese-Informationen der Hilfskassette identisch mit den Verschreibungs-Informationen sind, und eines Status, wenn sich die Lese-Informationen der Hilfskassette von den Verschreibungs-Informationen unterscheiden, um eine Warnung zu erzeugen und einen Verschreibungs-Fehler zu verhindern.

11. Vorrichtung zum Erfassen von Informationen einer Hilfskassette zum Zuführen von Medikamenten zu einer Kassette, die an einem Schubladenelement installiert ist, das in einem Sondertabletten-System vorhanden ist, das eingesetzt wird, wenn eine Hälfte einer Tablette oder ein Medikament zusammengestellt wird, das nicht in einem Behälter enthalten ist, wobei die Vorrichtung umfasst:
eine Anzeigeeinheit, die an einer vorgegebenen Position der Hilfskassette installiert ist, um Medikamenten-Informationen und Patienten-informationen optisch anzuzeigen; und
eine Einheit zum Verwalten der Anzeigeeinheit, die mit der Anzeigeeinheit über ein POS-System verbunden ist, um die Medikamenten-Informationen der in der Hilfskassette aufgenommenen Medikamente und die Patienten-Informationen zu der Anzeigeeinheit zu senden.

12. Vorrichtung nach Anspruch 11, wobei die Anzeigeeinheit umfasst:
eine Funkkommunikationseinheit, die Anzeige-Informationen, wie beispielsweise die Medikamenten-Informationen und die Patienten-Informationen, die von der Einheit zum Verwalten der Anzeigeeinheit gesendet werden, über Funkkommunikation empfängt und, wenn erforderlich, Bestätigungs-Informationen zu der Einheit zum Verwalten der Anzeigeeinheit sendet;
eine Signalverarbeitungseinrichtung zum Entfernen von Rauschen und zum Extrahieren eines reinen Datensignals aus einem durch die Funkkommunikationseinheit empfangenen Signal sowie zum Umwandeln des extrahierten reinen Datensignals in ein digitales Signal;
eine Steuereinrichtung, die Autorisierung/Analyse des durch die Signalverarbeitungseinrichtung verarbeiteten Signals durchführt und aktuell angezeigte Informationen so steuert, dass sie modifiziert werden, wenn mit der Autorisierung/Analyse festgestellt wird, dass das verarbeitete Signal ein Signal der Steuereinrichtung ist;
einen Speicher, der mit der Steuereinrichtung verbunden ist, um ein Programm für die Autorisierung/Analyse von Informationen und die angezeigten Informationen zu speichern;
eine Einrichtung zum Modifizieren von Informationen, die mit der Steuereinrichtung verbunden ist, um die angezeigten Informationen zu modifizieren; und
eine Anzeigeeinrichtung, die mit der Einrichtung zum Modifizieren von Informationen verbunden ist, um die Informationen der Hilfskassette entsprechend einem Ausgang von der Einrichtung zum Modifizieren von Informationen anzuzeigen.

13. Vorrichtung nach den Ansprüchen 11 oder 12, wobei die Anzeigeeinheit eine Preiskarte, auf der optisch anzuzeigende Informationen modifiziert werden können, eine Flüssigkristall-Anzeigeeinrichtung, eine flexible numerische Anzeigeeinrichtung oder eine Punktmatrix-Anzeigeeinrichtung umfasst.

14. Vorrichtung nach Anspruch 12, wobei die Steuereinrichtung umfasst:
ein Datenverarbeitungsmodul, das durch Verarbeiten der empfangenen Daten eine Identifizierungsnummer aus Daten heraustrennt;
ein Autorisierungsmodul, das die herausgetrennte Identifizierungsnummer mit einer in dem Speicher gespeicherten Identifizierungsnummer vergleicht, um die Autorisierung durchzuführen;
ein Steuermodul, das Speichern der empfangenen Daten über ein Speichermodul steuert und zu modifizierende Informationen ausgibt, wenn mit der Autorisierung festgestellt wird, dass die Daten Daten des Steuermoduls sind; und
ein Modul zum Ausgeben modifizierter Informationen, das die zu modifizierenden Medikamenten-Informationen und Patienten-Informationen in Reaktion auf die Steuerung des Steuermoduls ausgibt

15. Vorrichtung nach Anspruch 11, wobei die Einheit zum Verwalten der Anzeigeeinheit umfasst:
eine Eingabeeinheit, die aufzuzeichnende Eingabeinformationen und modifizierte Informationen der aufzuzeichnenden Informationen in die Anzeigeeinheit eingibt, die an der Hilfskassette angebracht ist;
eine Kommunikationseinheit, die Aufzeichnungsinformationen und die modifizierten Informationen der jeweiligen Anzeigeeinheiten in Echtzeit über die Kommunikation mit dem POS-System empfängt;
eine zentrale Verarbeitungseinheit, die mit der Eingabeeinheit und der Kommunikationseinheit verbunden ist, um die Anzeigeinformationen zu den jeweiligen Anzeigeeinheiten zu senden und das Senden der Informationen für die Modifizierung der anzuzeigenden Informationen steuert;
eine optische Anzeigeeinrichtung, die mit der zentralen Verarbeitungseinheit verbunden ist, um die zu einer speziellen Anzeigeeinheit zu sendenden Informationen optisch anzuzeigen; und
eine Funkkommunikationseinheit, die mit der zentralen Verarbeitungseinheit verbunden ist, um die Anzeige-Informationen über Funkkommunikation zu der Anzeigeeinheit zu senden.

## Revendications

1. Système de préparation de comprimés spéciaux, comprenant :
- un élément coulissant ;
- un plateau installé sur l'élément coulissant ;
- un plateau auxiliaire pour délivrer des médicaments au plateau installé sur l'élément coulissant ; et
- un appareil configuré pour détecter des informations de plateau auxiliaire, l'appareil comprenant :
- une étiquette d'identification radiofréquence, installée à une position prédéterminée du plateau auxiliaire, dans laquelle des informations de médicaments et des informations de patients sont enregistrées ;
- un lecteur d'informations d'identification radiofréquence configuré pour lire les informations enregistrées dans l'étiquette d'identification radiofréquence sans contact ;
- un terminal de gestion configuré pour comparer les informations de médicaments et les informations de patients lues par le lecteur d'informations d'identification radiofréquence à des informations de prescriptions fournies en entrée depuis l'extérieur, le terminal de gestion étant en outre configuré pour déterminer que le plateau auxiliaire est un plateau auxiliaire prédéterminé, lorsque les informations de médicaments et les informations de patients sont identiques aux informations de prescriptions, et configuré pour générer une alarme pour empêcher une erreur de prescription, lorsque les informations de médicaments et les informations de patients sont différentes des informations de prescriptions ; et
- un dispositif d'alarme connecté au terminal de gestion et configuré pour générer l'alarme pour empêcher l'erreur de prescription.

2. Système selon la revendication 1, dans lequel l'étiquette d'identification radiofréquence rend possible la modification des informations de médicaments et des informations de patients enregistrées sur celle-ci.

3. Système selon la revendication 1, dans lequel le lecteur d'informations d'identification radiofréquence comprend :
- un lecteur d'identification radiofréquence pour lire des données stockées dans l'étiquette d'identification radiofréquence sans contact ;
- une unité centrale de traitement pour commander une transmission des données lues par le lecteur d'identification radiofréquence ; et
- un émetteur-récepteur pour transmettre des informations de lecture d'identification radiofréquence délivrées en sortie de l'unité centrale de traitement à un dispositif externe en utilisant un protocole de communication prédéterminé.

4. Système selon les revendications 1 ou 3, dans lequel le lecteur d'informations d'identification radiofréquence est installé à une position prédéterminée de l'élément coulissant.

5. Système selon la revendication 1, dans lequel le terminal de gestion comprend :
- un émetteur-récepteur pour recevoir des informations de lecture de plateau auxiliaire transmises par le lecteur d'informations d'identification radiofréquence ;
- un contrôleur pour comparer les informations de lecture de plateau auxiliaire transmises par l'émetteur-récepteur aux informations de prescriptions précédemment stockées et générer l'alarme pour empêcher l'erreur de prescription lorsque les informations de lecture de plateau auxiliaire sont différentes des informations de prescriptions précédemment stockées ;
- un afficheur pour afficher les informations de prescriptions et les informations de lecture de plateau auxiliaire sur un écran ;
- une mémoire dans laquelle les informations de prescriptions et un programme de comparaison d'informations et de génération de l'alarme sont stockés ; et
- une unité d'alarme pour générer un signal d'alarme correspondant à un signal de commande, lorsque le signal de commande est délivré en sortie du contrôleur, et transmettre le signal d'alarme au dispositif d'alarme.

6. Système selon la revendication 5, dans lequel le contrôleur comprend :
- une interface pour réaliser un interfaçage entre l'émetteur-récepteur, l'afficheur, l'unité d'alarme, la mémoire et un dispositif externe pour interfacer des données ;
- un comparateur d'informations pour comparer les informations de prescriptions aux informations de lecture de plateau auxiliaire ;
- un générateur d'alarme pour générer un signal de commande d'alarme, lorsque les informations de prescriptions sont différentes des informations de lecture de plateau auxiliaire, en résultat de la comparaison effectuée par le comparateur d'informations ; et
- un contrôleur d'affichage pour commander l'affichage des informations de prescriptions et des informations de lecture de plateau auxiliaire.

7. Système selon la revendication 6, dans lequel le dispositif d'alarme est installé à une position prédéterminée de l'élément coulissant.

8. Système selon les revendications 1 ou 7, dans lequel le dispositif d'alarme comprend une diode électroluminescente pour émettre de la lumière suivant un signal d'alarme reçu en entrée, de façon à générer une alarme visuelle prévenant de l'erreur de prescription.

9. Système selon les revendications 1 ou 7, dans lequel le dispositif d'alarme comprend un dispositif produisant une alarme prévenant de l'erreur de prescription en générant un son d'alarme suivant un signal d'alarme audible reçu en entrée.

10. Procédé de détection d'informations d'un plateau auxiliaire pour délivrer des médicaments à un plateau installé sur un élément coulissant prévu dans un système de préparation de comprimés spéciaux, utilisé lors de la préparation d'une moitié d'un comprimé ou d'un médicament non contenu dans une cassette, le procédé comprenant les étapes consistant à :
- effectuer une initialisation d'un appareil lorsqu'une alimentation électrique est fournie et vérifier si des informations de prescriptions sont reçues ;
- stocker les informations de prescriptions reçues dans la mémoire lorsque les informations de prescriptions sont reçues en résultat de la vérification ;
- déterminer si des informations de lecture de plateau auxiliaire sont reçues après le stockage des informations de prescriptions ;
- comparer les informations de lecture de plateau auxiliaire reçues aux informations de prescriptions stockées lorsque les informations de lecture de plateau auxiliaire sont reçues ; et
- afficher un état, lorsque les informations de lecture de plateau auxiliaire sont identiques aux informations de prescriptions, ou un état, lorsque les informations de lecture de plateau auxiliaire sont différentes des informations de prescriptions, de manière à générer une alarme pour empêcher une erreur de prescription.

11. Appareil de détection d'informations d'un plateau auxiliaire pour délivrer des médicaments à un plateau installé sur un élément coulissant prévu dans un système de préparation de comprimés spéciaux, utilisé lors de la préparation d'une moitié d'un comprimé ou d'un médicament non contenu dans une cassette, l'appareil comprenant :
- une unité d'indication installée à une position prédéterminée du plateau auxiliaire pour indiquer visuellement des informations de médicaments et des informations de patients ; et
- une unité de gestion d'unité d'indication connectée à l'unité d'indication par un système POS afin de transmettre les informations de médicaments, des médicaments disposés dans le plateau auxiliaire, et les informations de patients à l'unité d'indication.

12. Appareil selon la revendication 11, dans lequel l'unité d'indication comprend :
- une unité de radiocommunication pour recevoir des informations d'indication, telles que les informations de médicaments et les informations de patients qui sont transmises depuis l'unité de gestion d'unité d'indication par radiocommunication, et transmettre des informations de confirmation à l'unité de gestion d'unité d'indication suivant les besoins ;
- un processeur de signal pour éliminer le bruit, extraire un signal de données pur, à partir du signal reçu par l'unité de radiocommunication, et convertir le signal de données pur extrait en un signal numérique ;
- un contrôleur pour effectuer une habilitation/analyse du signal traité par le processeur de signal et commander une modification d'informations présentement indiquées lorsqu'il est déterminé que le signal traité est un signal du contrôleur en résultat de l'habilitation/analyse ;
- une mémoire connectée au contrôleur pour stocker un programme pour l'habilitation/analyse d'informations et les informations indiquées ;
- un modificateur d'informations connecté au contrôleur pour modifier les informations indiquées ; et
- un indicateur connecté au modificateur d'informations pour indiquer les informations de plateau auxiliaire suivant une sortie en provenance du modificateur d'informations.

13. Appareil selon les revendications 11 ou 12, dans lequel l'unité d'indication comprend un dispositif parmi une carte de réduction, dans laquelle des informations à afficher peuvent être modifiées, un afficheur à cristaux liquides, un afficheur numérique souple et un afficheur à matrice de points.

14. Appareil selon la revendication 12, dans lequel le contrôleur comprend :
- un module de traitement de données pour séparer un numéro d'identification de données en traitant les données reçues ;
- un module d'habilitation qui compare le numéro d'identification séparé à un numéro d'identification stocké dans la mémoire pour effectuer l'habilitation ;
- un module de commande pour commander le stockage des données reçues par un module de stockage et délivrer en sortie des informations à modifier, lorsqu'il est déterminé que les données sont des données du module de commande en résultat de l'habilitation ; et
- un module de sortie d'informations modifiées pour délivrer en sortie les informations de médicaments et les informations de patients à modifier en réponse à la commande du module de commande.

15. Appareil selon la revendication 11, dans lequel l'unité de gestion d'unité d'indication comprend :
- une unité d'entrée pour délivrer en entrée des informations à enregistrer et des informations modifiées des informations à enregistrer à l'unité d'indication liée au plateau auxiliaire ;
- une unité de communication pour recevoir des informations d'enregistrement et les informations modifiées des unités d'indication respectives en temps réel par la communication avec le système POS ;
- une unité centrale de traitement connectée à l'unité d'entrée et à l'unité de communication pour transmettre les informations d'indication aux unités d'indication respectives et commander la transmission des informations pour la modification des informations à indiquer ;
- un afficheur connecté à l'unité centrale de traitement pour afficher les informations à transmettre à une unité d'indication spécifique ; et
- une unité de radiocommunication connectée à l'unité centrale de traitement pour transmettre les informations d'indication à l'unité d'indication via une radiocommunication.
